# EUROPEAN PATENT SPECIFICATION

(11) **EP 2 123 627 B1**
(45) Date of publication and mention of the grant of the patent: **20.11.2013**
(21) Application number: 07707070.4
(22) Date of filing: 19.01.2007
(51) Int. Cl.: C07C 213/02, C07C 217/86, C07C 219/34, C07D 213/73, C07B 61/00

(54) **PROCESS FOR PRODUCING AROMATIC AMINE HAVING ARALKYLOXY OR HETEROARALKYLOXY GROUP**
VERFAHREN ZUR HERSTELLUNG EINES AROMATISCHEN AMINS MIT EINER ARALKYLOXY- ODER HETEROARALKYLOXYGRUPPE
PROCÉDÉ DE PRODUCTION D'AMINE AROMATIQUE AYANT UN GROUPE ARALKYLOXY OU HÉTÉROARALKYLOXY

(43) Date of publication of application: 25.11.2009
(73) Proprietor: Ube Industries, Ltd., Ube-shi Yamaguchi 755-8633 (JP)
(72) Inventor: NISHINO, Shigeyoshi, Yamaguchi, 755-8633 (JP); SHIMA, Hidetaka, Yamaguchi, 755-8633 (JP); ODA, Hiroyuki, Yamaguchi, 755-8633 (JP)
(74) Representative: HOFFMANN EITLE
(86) International application number: PCT/JP2007/050782
(87) International publication number: WO 2008/087736

(56) References cited:
- EP-A1- 1 437 344
- WO-A1-2004/054985
- WO-A1-2004/105765
- WO-A1-2004/105765
- WO-A1-2005/058318
- WO-A2-2007/030361
- JP-A- 51 043 731
- JP-A- 2001 503 772
- JP-A- 2003 286 263
- JP-A- 2006 519 232
- JP-A- 2007 051 128
- US-A1- 2005 267 106
- TIANSHENG WANG, ALFRED S. LUI, IAN S. CLOUDSDALE: "A Novel Route to Pyrrolo[2,1-c][1,4]benzodiazepin-5-ones. Formal Total Synthesis of (+-)-DC-81", ORGANIC LETTERS, vol. 1, 1999, pages 1835-1837,
- ATSUKO NOSE AND TADAHIRO KUDO: "Reduction of Sodium Borohydride-Transition Metal Salt Systems I. Reduction of Aromatic Nitro Compounds with the Sodium Borohydride-Nickelous Chloride System", CHEMICAL AND PHARMACEUTICAL BULLETIN, vol. 29, 1981, pages 1159-1161,

## Description

### Technical Field

The present invention relates to a process for producing an aromatic amine compound having an aralkyloxy or heteroaralkyloxy group, in which an aromatic nitro compound having an aralkyloxy or heteroaralkyloxy group is reacted with hydrogen while preventing the removal of aralkyloxy or heteroaralkyloxy group and keeping stable the other substituent on the aromatic group so that the substituent does not take part in the reaction, to reduce the nitro group. The aromatic amine compound having an aralkyloxy or heteroaralkyloxy group is a compound which is useful as, for example, a raw material for medical products or agricultural chemicals or an intermediate in the synthesis of them.

### Background Art

As a conventional method for producing an aromatic amine compound, such as aniline, having an aralkyloxy or heteroaralkyloxy group, in which an aromatic nitro compound, such as nitrobenzene, having an aralkyloxy or heteroaralkyloxy group is reacted with hydrogen while preventing the removal of aralkyloxy or heteroaralkyloxy group, to reduce the nitro group, for example, the following methods are disclosed:
(1) a method in which 4-benzyloxy-5-methoxy-2-nitrobenzoic acid is reacted in methanol in the presence of stannous chloride to obtain 4-benzyloxy-5-methoxy-2-aminobenzoic acid (see, for example, non-patent document 1);
(2) a method in which benzyl 4-benzyloxy-5-methoxy-2-nitrobenzoate is reacted with stannous chloride in ethyl acetate (see, for example, non-patent document 2); and
(3) a method in which 4-benzyloxy-5-methoxy-2-nitrobenzoic acid is reacted in an ethanol/acetic acid/water mixed solvent in the presence of iron and hydrochloric acid to obtain 4-benzyloxy-5-methoxy-2-aminobenzoic acid (see, for example, non-patent document 3).

However, each of the above methods has a disadvantage that the reaction, which is of a homogeneous system, requires a cumbersome operation for removing the remaining metals or by-products formed during the reaction from the reaction mixture, so that the reaction cannot be performed by a continuous process. That is, the conventional methods for producing an aromatic amine compound having an aralkyloxy or heteroaralkyloxy group are industrially disadvantageous. Therefore, there has been desired the development of an industrially practical method with which the product, by-products, catalyst, and others can be easily recovered, and the reaction can be performed by a continuous process.

Non-patent document 1: J. Org. Chem., 66 (8), 2881 (2001)
Non-patent document 2: J. Med. Chem., 45 (17), 3772 (2002)
Non-patent document 3: Tetrahedron, 51 (19), 5617 (1995)

Tiansheng Wang et al., Organic Letters, vol. 1, 1999, pages 1835-1837 report on a synthesis of pyrrolo[2,1-c][1,4]benzo-diazepine-5-ones, wherein 4-benzyloxy-5-methoxy-2-nitrobenzoic acid methyl ester is reduced to the corresponding 2-amino derivative with sodium borohydride using NiCl₂ as a catalyst.

### DISCLOSURE OF THE INVENTION

### Problems to be Solved by the Invention

Accordingly, the problem of the present invention is to solve the above-mentioned problems and to provide a process for producing an aromatic amine compound having an aralkyloxy or heteroaralkyloxy group, in which an aromatic nitro compound having an aralkyloxy or heteroaralkyloxy group is reacted with hydrogen under mild conditions in accordance with a simple procedure while preventing the removal of aralkyl or heteroaralkyl group and keeping stable the other substituent on the aromatic group so that the substituent does not take part in the reaction, to reduce the nitro group.

### Means for Solving the Problems

The present invention is directed to a process for producing an aromatic amine compound, which comprises reacting an aromatic nitro compound represented by the general formula (I): 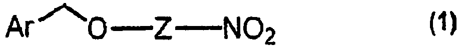 wherein Ar represents an aryl group or heteroaryl group which may have a substituent, and Z represents a divalent aromatic group which may have a substituent,
with a hydrogen gas in the presence of a metal catalyst selected from the group consisting of platinum/carbon, platinum-sulfur/carbon, palladium-platinum/carbon, platinum oxide, platinum/alumina and rhodium/carbon to obtain an aromatic amine compound represented by the general formula (2): 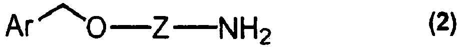 wherein Ar and Z have the same meanings as defined above, and
wherein the compound of the general formula (1) is 4-benzyloxy-5-methoxy-2-nitrobenzoic acid alkyl ester.

### Effect of the Invention

In the process of the present invention, an aromatic nitro compound having a benzyloxy group is reacted with hydrogen under mild conditions in accordance with a simple procedure, while preventing the removal of benzyl group and keeping stable the other substituent on the aromatic group so that the substituent does not take part in the reaction, to selectively reduce only the nitro group, thus producing an aromatic amine compound having a benzyloxy group.

### BEST MODE FOR CARRYING OUT THE INVENTION

In the process of the present invention, the alkyl group means a linear or branched aliphatic hydrocarbon group having 1 to 12 carbon atoms, preferably I to 8 carbon atoms.

In the process of the present invention, the nitro group on the divalent aromatic group represented by Z is selectively reduced by hydrogen. Therefore, even when the highly reactive substituent shown above is present on the divalent aromatic group, the substituent does not take part in the reaction and remains stable, so that the reaction in the process of the present invention rapidly proceeds.

With respect to the metal catalyst used in the reaction in the present invention, there is no particular limitation as long as it is a hydrogenation catalyst which can achieve a reaction of the nitro group in the compound of the formula (I) with hydrogen to reduce the nitro group to an amino group without the removal of benzyl group due to the removal of Ar-CH₂-O- group. With respect to the metal catalyst usable in the reaction in the present invention, specific examples include platinum/carbon, platinum-sulfur/carbon, palladium-platinum/carbon, platinum oxide, platinum/alumina, and rhodium/carbon, and platinum-sulfur/carbon is preferably used. Of these, platinum-sulfur/carbon (e.g., platinum sulfided carbon), which is obtained by treating platinum with sulfur so as to control the catalytic activity, can be especially preferably used. These metal catalysts can be used alone or in combination, and may be hydrous or anhydrous catalysts.

The amount of the metal catalyst used is preferably 0.1 to 1,000 mg, further preferably 0.5 to 500 mg, in terms of a metal atom, relative to 1 g of the aromatic nitro compound of the formula (1).

The amount of the hydrogen used in the reaction in the present invention is preferably 2 to 50 mol, further preferably 2 to 10 mol, relative to 1 mol of the aromatic nitro compound of the formula (1).

The reaction in the present invention can be conducted either in a solvent or in the absence of a solvent. When a solvent is used in the reaction, there is no particular limitation to the solvent as long as it does not inhibit the reaction, and examples of solvents include water; alcohols, such as methanol, ethanol, isopropyl alcohol, n-butyl alcohol, and t-butyl alcohol; carboxylic acid esters, such as methyl acetate, ethyl acetate, and methyl propionate; aromatic hydrocarbons, such as benzene, toluene, xylene, and mesitylene; and ethers, such as diethyl ether, dimethoxyethane, tetrahydrofuran, dioxane, and diisopropyl ether. An ester or ether is preferably used, and a carboxylic acid ester, such as butyl acetate, or an ether, such as dimethoxyethane, is further preferably used. These solvents can be used alone or in combination.

The amount of the solvent used is appropriately selected depending on the uniformity or stirring properties of the reaction mixture, but it is preferably 0 to 100 g, further preferably 0 to 30 g, relative to 1 g of the aromatic nitro compound of the formula (1).

The reaction in the present invention is conducted in accordance with a method in which, for example, the aromatic nitro compound of the formula (1), a metal catalyst, and a solvent are mixed together and stirred in the presence of hydrogen gas (which may be diluted with inert gas). In this instance, the reaction temperature is preferably 0 to 300°C, further preferably 20 to 200°C, and the reaction pressure is preferably 0.1 to 10 MPa, further preferably 0.1 to 2 MPa.

An aromatic amine compound of the formula (2) is obtained by the reaction in the present invention, and, after completion of the reaction, the obtained aromatic amine compound is isolated and purified by a method generally used, such as neutralization, extraction, filtration, concentration, distillation, recrystallization, crystallization, or column chromatography.

A 4-benzyloxy-5-methoxy-2-aminobenzoic acid compound, which is a representative compound of the aromatic amine compound of the formula (2) obtained by the process of the present invention, can be produced from a 4-benzyloxy-5-methoxy-2-nitrobenzoic acid compound, such as 4-benzyloxy-5-methoxy-2-nitrobenzoic acid alkyl ester. The 4-benzyloxy-5-methoxy-2-aminobenzoic acid compound is reacted with, for example, an orthoformic acid ester in the presence of ammonium carboxylate, forming a 6-methoxy-7-benzyloxyquinazolin-4-one compound, which is useful as an intermediate compound for medical products or agricultural chemicals (described below in Reference Example 1).

### Examples

Hereinbelow, the present invention will be described in more detail with reference to the following Examples, which should not be construed as limiting the scope of the present invention.

### Example 1 (Synthesis of 2-benzyloxyaniline)

Into a glass vessel having an internal volume of 100 ml and equipped with a stirrer, a thermometer, a reflux condenser, and a balloon filled with hydrogen gas were placed 3.0 g (13.09 mmol) of 2-benzyloxynitrobenzene, 0.15 g of 3% by mass platinum sulfided carbon powder (50% moisture content) (manufactured by N.E. CHEMCAT CORPORATION), and 30 ml of butyl acetate, and the resultant mixture was reacted in a hydrogen gas atmosphere at 60°C for 3 hours with stirring. After completion of the reaction, the reaction mixture was filtered, and the resultant filtrate was concentrated under reduced pressure. The concentrate was purified by silica gel column chromatography (eluent; hexane:ethyl acetate = 4:1 (volume ratio)) to obtain 2.46 g of 2-benzyloxyaniline as a brown liquid (isolation yield: 94.6%).
Values of physical properties for the obtained 2-benzyloxyaniline are as follows.

¹H-NMR(CDCl₃, δ(ppm)); 3.85(2H,brs), 5.11(2H,s), 6.70-6.90(3H,m), 7.32-7.48(4H,m)
CI-MS(m/e); 200(M+)

### Example 2 (Synthesis of 3-benzyloxyaniline)

A reaction was conducted in the same manner as in Example 1 except that the 2-benzyloxynitrobenzene was changed to 3-benzyloxynitrobenzene. As a result, 1.80 g of 3-benzyloxyaniline as a beige solid was obtained (isolation yield: 69.2%).
Values of physical properties for the obtained 3-benzyloxyaniline are as follows.

¹H-NMR(CDCl₃, δ(ppm)); 3.66(2H,brs), 5.04(2H,s), 6.31-6.44(3H,m), 7.05-7.11(1H,m), 7.30-7.46(5H,m)
CI-MS(m/e); 200(M+)

### Example 3 (Synthesis of 4-benzyloxyaniline)

A reaction was conducted in the same manner as in Example 1 except that the 2-benzyloxynitrobenzene was changed to 4-benzyloxynitrobenzene. As a result, 2.29 g of 4-benzyloxyaniline as a brown solid was obtained (isolation yield: 88.0%).
Values of physical properties for the obtained 4-benzyloxyaniline are as follows.

¹H-NMR(CDCl₃, δ(ppm)); 3.44(2H,brs), 5.01(2H,s), 6.63-6.68(2H,m), 6.81-6.86(2H,m), 7.30-6.68(5H,m)
CI-MS(mle); 200(M+)

### Example 4 {Synthesis of 4-(3-fluorobenzyloxy)-3-chloroaniline}

Into a glass vessel having an internal volume of 100 ml and equipped with a stirrer, a thermometer, a reflux condenser, and a balloon filled with hydrogen gas were placed 3.0 g (10.65 mmol) of 4-(3-fluorobenzyloxy)-3-chloronitrobenzene, 0.15 g of 3% by mass platinum sulfided carbon powder (50% moisture content) (manufactured by N.E. CHEMCAT CORPORATION), and 30 ml of butyl acetate, and the resultant mixture was reacted in a hydrogen gas atmosphere at 60°C for 3 hours with stirring. After completion of the reaction, the reaction mixture was filtered, and the resultant filtrate was concentrated under reduced pressure. The concentrate was purified by silica gel column chromatography (eluent; hexane:ethyl acetate = 4:1 (volume ratio)) to obtain 2.36 g of 4-(3-fluorobenzyloxy)-3-chloroaniline as a brown solid (isolation yield: 88.0%).
Values of physical properties for the obtained 4-(3-fluorobenzyloxy)-3-chloroaniline are as follows.

¹H-NMR(CDCl₃, δ(ppm)); 3.51 (2H,brs), 5.04(2H,s), 6.49-6.53(1H,m), 6.77-6.80(2H,m), 6.97-7.04(1H,m), 7.19-7.23(2H,m), 7.31-7.38(1H,m)
CI-MS(m/e); 252(M+)

### Example 5 (Synthesis of 3-amino-2-benzyloxypyridine)

Into a glass vessel having an internal volume of 100 ml and equipped with a stirrer, a thermometer, and a reflux condenser were placed 2.0 g (8.68 mmol) of 3-nitro-2-benzyloxypyridine, 0.10 g of 3% by mass platinum sulfided carbon powder (50% moisture content) (manufactured by N.E. CHEMCAT CORPORATION), and 20 ml of butyl acetate, and the resultant mixture was reacted in a hydrogen gas atmosphere at 60°C for 3 hours with stirring. After completion of the reaction, the reaction mixture was filtered, and the resultant filtrate was concentrated under reduced pressure. The concentrate was purified by silica gel column chromatography (eluent; hexane:ethyl acetate = 1:1 (volume ratio)) to obtain 0.80 g of 3-amino-2-benzyloxypyridine as a brown solid (isolation yield: 46.2%).
Values of physical properties for the obtained 3-amino-2-benzyloxypyridine are as follows.

¹H-NMR(CDCl₃, δ(ppm)); 4.28(2H,brs), 5.16(2H,s), 6.01-6.06(1H,m), 6.49-6.52(1H,m), 6.70-6.73(1H,m), 7.24-7.36(5H,m)
CI-MS(m/e); 201(M+)

### Example 6 (Synthesis of methyl 2-amino-4-benzyloxy-5-methoxybenzoate)

Into a glass vessel having an internal volume of 20 ml and equipped with a stirrer, a thermometer, a reflux condenser, and a balloon filled with hydrogen gas were placed 4.0 g (12.6 mmol) of methyl 4-benzyloxy-5-methoxy-2-nitrobenzoate, 0.2 g of 3% by mass platinum sulfided carbon powder (60% moisture content) (manufactured by N.E. CHEMCAT CORPORATION), and 20 ml of dimethoxyethane, and the resultant mixture was reacted in a hydrogen gas atmosphere at room temperature for 7 hours with stirring. After completion of the reaction, the reaction mixture was allowed to stand for 10 days and then filtered, and the reaction mixture was concentrated under reduced pressure to obtain 3.17 g of methyl 2-amino-4-benzyloxy-5-methoxybenzoate as a pale brown solid (isolation yield: 87%).
Values of physical properties for the obtained methyl 2-amino-4-benzyloxy-5-methoxybenzoate are as follows.

¹H-NMR(CDCl₃, δ(ppm)); 3.81(3H,s), 3.83(3H,s), 5.09(2H,s), 5.37(2H,brs), 6.15(1H,s), 7.31-7.41(6H,m)
CI-MS(m/e); 287(M+)

### Example 7 (Synthesis of methyl 2-amino-4-benzyloxy-5-methoxybenzoate)

A reaction was conducted in the same manner as in Example 6 except that 3% by mass platinum sulfided carbon powder was changed to 5% by mass platinum/alumina. As a result, 2.17 g of methyl 2-amino-4-benzyloxy-5-methoxybenzoate was formed (reaction yield: 54.6%).

### Example 8 (Synthesis of methyl 2-amino-4-benzyloxy-5-methoxybenzoate)

A reaction was conducted in the same manner as in Example 6 except that 3% by mass platinum sulfided carbon powder was changed to 5% by mass rhodium/carbon. As a result, 3.15 g of methyl 2-amino-4-benzyloxy-5-methoxybenzoate was formed (reaction yield: 79.2%).

### Reference Example 1 (Synthesis of 6-methoxy-7-benzyloxyquinazolin-4-one)

Into a glass vessel having an internal volume of 20 ml and equipped with a stirrer, a thermometer, and a reflux condenser were placed 2.0 g (6.96 mmol) of methyl 2-amino-4-benzyloxy-5-methoxybenzoate, 2.15 g (27.8 mmol) of ammonium acetate, 2.95 g (27.8 mmol) of methyl orthoformate, and 8 ml of methanol, and the resultant mixture was reacted at 60 to 70°C with stirring. After completion of the reaction, the reaction mixture was filtered, and then the filtrate was dried to obtain 1.74 g of 6-methoxy-7-benzyloxyquinazolin-4-one as a pale yellow solid (isolation yield: 88%).
Values of physical properties for the obtained 6-methoxy-7-benzyloxyquinazolin-4-one are as follows.

¹H-NMR(CDCl₃, δ(ppm)); 3.87(3H,s), 5.26(2H,s), 7.23(1H,s), 7.33-7.50(6H,m), 7.97(1H,s), 12.04(1H,brs)
CI-MS(m/e); 283(M+1)

### INDUSTRIAL APPLICABILITY

The present invention is directed to a process for producing an aromatic amine having an aralkyloxy or heteroaralkyloxy group, in which an aromatic nitro compound having an aralkyloxy or heteroaralkyloxy group is reacted with hydrogen under mild conditions in accordance with a simple procedure while preventing the removal of aralkyl or heteroaralkyl group and keeping stable the other substituent on the aromatic group so that the substituent does not take part in the reaction, to selectively reduce only the nitro group. The aromatic amine having an aralkyloxy or heteroaralkyloxy group is a compound which is useful as, for example, a raw material for medical products or agricultural chemicals or an intermediate in the synthesis of them.

## Claims

1. A process for producing an aromatic amine compound,
which comprises reacting an aromatic nitro compound represented by the general formula (1): 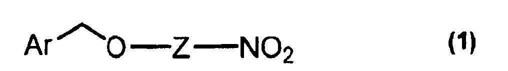 wherein Ar represents an aryl group or heteroaryl group which may have a substituent, and Z represents a divalent aromatic group which may have a substituent,
with hydrogen gas in the presence of a metal catalyst selected from the group consisting of platinum/carbon, platinum-sulfur/carbon, palladium-platinum/carbon, platinum oxide, platinum/alumina and rhodium/carbon to obtain an aromatic amine compound represented by the general formula (2): 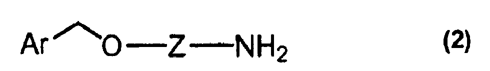 wherein Ar and Z have the same meanings as defined above, and
wherein the compound of the general formula (1) is 4-benzyloxy-5-methoxy-2-nitrobenzoic acid alkyl ester.

2. The process for producing an aromatic amine compound according to claim 1, wherein the metal catalyst is supported on a carrier.

3. The method for producing an aromatic amine compound according to claim 1, wherein the metal catalyst is platinum-sulfur/carbon.

4. The process for producing an aromatic amine compound according to claim 3, wherein the metal catalyst is platinum-sulfur/carbon obtained by treating platinum with sulfur.

5. The process for producing an aromatic amine compound according to claim 1, wherein the process is carried out at a temperature of 20 to 200°C.

6. The process for producing an aromatic amine compound according to claim 1, wherein an amount of the metal catalyst is 0.1 to 1,000 mg, preferably 0.5 to 500 mg, in terms of a metal atom, relative to 1 g of the aromatic nitro compound of the formula (1).

7. The process for producing an aromatic amine compound according to claim 1, wherein the process is carried out in a solvent selected from an ester and an ether.

8. The process for producing an aromatic amine compound according to claim 7, wherein the ester is selected from a carboxylic acid ester.

9. The process for producing an aromatic amine compound according to claim 1, wherein an amount of the hydrogen gas is 2 to 50 mol, preferably 2 to 10 mol, relative to 1 mol of the aromatic nitro compound of the formula (1).

10. The process for producing an aromatic amine compound according to claim 1, wherein the process is carried out under a reaction pressure of 0.1 to 10 MPa, preferably 0.1 to 2 MPa.

## Patentansprüche

1. Verfahren zur Herstellung einer aromatischen Aminverbindung,
die das Umsetzen einer aromatischen Nitroverbindung der allgemeinen Formel (1): 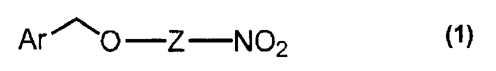 worin Ar eine Arylgruppe oder Heteroarylgruppe darstellt, die einen Substituenten aufweisen kann und Z eine zweiwertige aromatische Gruppe darstellt, die einen Substituenten aufweisen kann,
mit Wasserstoffgas in Gegenwart eines Metallkatalysators, ausgewählt aus der Gruppe bestehend aus Platin/Kohlenstoff, Platin-Schwefel/Kohlenstoff, Palladium-Platin/Kohlenstoff, Platinoxid, Platin/Aluminiumoxid und Rhodium/Kohlenstoff, umfasst, um eine aromatische Aminverbindung der allgemeinen Formel (2) zu erhalten: 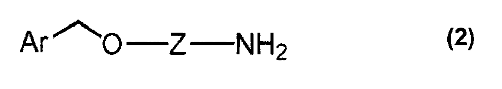 worin Ar und Z die gleichen Bedeutungen haben wie oben definiert, und
wobei die Verbindung der allgemeinen Formel (1) 4-Benzyloxy-5-methoxy-2-nitrobenzoesäurealkylester ist.

2. Verfahren zur Herstellung einer aromatischen Aminverbindung gemäss Anspruch 1, wobei der Metallkatalysator auf einem Träger getragen wird.

3. Verfahren zur Herstellung einer aromatischen Aminverbindung gemäss Anspruch 1, wobei der Metallkatalysator Platin-Schwefel/Kohlenstoff ist.

4. Verfahren zur Herstellung einer aromatischen Aminverbindung gemäss Anspruch 3, wobei der Metallkatalysator Platin-Schwefel/Kohlenstoff ist, der durch Behandeln von Platin mit Schwefel erhalten wird.

5. Verfahren zur Herstellung einer aromatischen Aminverbindung gemäss Anspruch 1, wobei das Verfahren bei einer Temperatur von 20 bis 200°C durchgeführt wird.

6. Verfahren zur Herstellung einer aromatischen Aminverbindung gemäss Anspruch 1, wobei die Menge des Metallkatalysators 0,1 bis 1.000 mg, vorzugsweise 0,5 bis 500 mg, bezogen auf ein Metallatom, im Verhältnis zu 1 g der aromatischen Nitroverbindung der Formel (1), beträgt.

7. Verfahren zur Herstellung einer aromatischen Aminverbindung gemäss Anspruch 1, wobei das Verfahren in einem Lösungsmittel, ausgewählt aus einem Ester und einem Ether, durchgeführt wird.

8. Verfahren zur Herstellung einer aromatischen Aminverbindung gemäss Anspruch 7, wobei der Ester aus einem Carbonsäureester ausgewählt ist.

9. Verfahren zur Herstellung einer aromatischen Aminverbindung gemäss Anspruch 1, wobei die Menge an Wasserstoffgas 2 bis 50 mol, vorzugsweise 2 bis 10 mol, im Verhältnis zu 1 mol der aromatischen Nitroverbindung der Formel (1), beträgt.

10. Verfahren zur Herstellung einer aromatischen Aminverbindung gemäss Anspruch 1, wobei das Verfahren unter einem Reaktionsdruck von 0,1 bis 10 MPa, vorzugsweise 0,1 bis 2 MPa, durchgeführt wird.

## Revendications

1. Procédé de production d'un composé amine aromatique,
qui comprend la réaction d'un composé nitro aromatique représenté par la formule générale (1) : 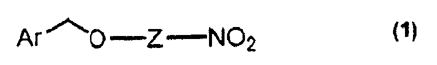 dans laquelle Ar représente un groupe aryle ou un groupe hétéroaryle qui peut porter un substituant, et Z représente un groupe aromatique divalent qui peut porter un substituant,
avec de l'hydrogène gazeux en présence d'un catalyseur métallique choisi dans le groupe constitué du platine/charbon, du platine-soufre/charbon, du palladium-platine/charbon, de l'oxyde de platine, du platine/alumine et du rhodium/charbon, pour donner un composé amine aromatique représenté par la formule générale (2) : 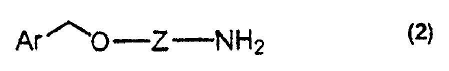 dans laquelle Ar et Z ont les mêmes significations que celles définies ci-dessus, et dans laquelle le composé de formule générale (1) est un alkylester d'acide 4-benzyloxy-5-méthoxy-2-nitrobenzoïque.

2. Procédé de production d'un composé amine aromatique selon la revendication 1, dans lequel le catalyseur métallique est supporté sur un support.

3. Procédé de production d'un composé amine aromatique selon la revendication 1, dans lequel le catalyseur métallique est du platine-soufre/charbon.

4. Procédé de production d'un composé amine aromatique selon la revendication 3, dans lequel le catalyseur métallique est du platine-soufre/charbon obtenu par traitement de platine avec du soufre.

5. Procédé de production d'un composé amine aromatique selon la revendication 1, dans lequel le procédé est réalisé à une température de 20 à 200°C.

6. Procédé de production d'un composé amine aromatique selon la revendication 1, dans lequel la quantité de catalyseur métallique est de 0,1 à 1 000 mg, de préférence de 0,5 à 500 mg, en termes d'atome de métal, par gramme du composé nitro aromatique de formule (1).

7. Procédé de production d'un composé amine aromatique selon la revendication 1, dans lequel le procédé est réalisé dans un solvant choisi parmi un ester et un éther.

8. Procédé de production d'un composé amine aromatique selon la revendication 7, dans lequel l'ester est choisi parmi un ester d'acide carboxylique.

9. Procédé de production d'un composé amine aromatique selon la revendication 1, dans lequel la quantité d'hydrogène gazeux est de 2 à 50 mol, de préférence de 2 à 10 mol, par mole du composé nitro aromatique de formule (1).

10. Procédé de production d'un composé amine aromatique selon la revendication 1, dans lequel le procédé est réalisé sous une pression réactionnelle de 0,1 à 10 MPa, de préférence de 0,1 à 2 MPa.
